**Europäisches Patentamt**

**European Patent Office**

**Office européen des brevets**

(19)

(11) Numéro de publication : **0 347 448 B1**

(12)

# FASCICULE DE BREVET EUROPEEN

(45) Date de publication du fascicule du brevet :
**20.05.92 Bulletin 92/21**

(51) Int. Cl.$^5$ : **A61K 35/02,** A61K 33/00,
// (A61K35/02, 33:00, 33:42,
31:19)

(21) Numéro de dépôt : **89900834.6**

(22) Date de dépôt : **21.12.88**

(86) Numéro de dépôt international :
**PCT/FR88/00626**

(87) Numéro de publication internationale :
**WO 89/06133 13.07.89 Gazette 89/15**

(54) **PREPARATION AQUEUSE A BASE DE SELS MINERAUX ET D'ACIDE ACETIQUE POUR LA PREVENTION ET LE TRAITEMENT DES MALADIES VIRALES.**

(30) Priorité : **08.01.88 FR 8800119**

(73) Titulaire : **Commin, Alix-Roland
2, place Gabriel Péri
F-94400 Vitry sur Seine (FR)**

(43) Date de publication de la demande :
**27.12.89 Bulletin 89/52**

(72) Inventeur : **Commin, Alix-Roland
2, place Gabriel Péri
F-94400 Vitry sur Seine (FR)**

(45) Mention de la délivrance du brevet :
**20.05.92 Bulletin 92/21**

(84) Etats contractants désignés :
**AT BE CH DE FR GB IT LI LU NL SE**

EP 0 347 448 B1

Jouve, 18, rue Saint-Denis, 75001 PARIS

## Description

La présente invention propose, pour la prévention et le traitement des maladies virales, une solution aqueuse contenant, d'une part, de 15 à 20 g/l d'un mélange de sels formés à partir des cations sodium, magnésium, calcium, potassium, strontium et bore et des anions chlore, sulfate, carbonate et bicarbonate et, d'autre part, de 3 à 4% d'acide acétique.

Il est entendu que l'acide acétique peut donner lui aussi des sels avec les métaux alcalins et alcalino-terreux précités.

De préférence, la solution aqueuse renferme, en outre, des composés minéraux azotés choisis entre les nitrites, les nitrates et les sels ammoniacaux.

Avantageusement, la solution aqueuse renferme, en outre, des phosphates et de la silice.

Dans une forme d'exécution pratique de l'invention, la solution aqueuse est préparée à partie d'eau de mer et de vinaigre blanc, matières premières qui apportent les différents constituants voulus pour la solution.

Dans ce cas, la solution résultante est stérilisée par filtration à froid sur bougie poreuse.

La solution selon l'invention peut être administrée par voie parentérale, notamment par injection intraveineuse ou intramusculaire, auquel cas elle sera convenablement tamponnée et/ou diluée pour être isotonique au plasma sanguin.

La solution peut également être administrée sous forme de lavement intestinal.

Le médicament résultant convient à la prévention et au traitement des maladies virales.

## Revendications

1. Solution aqueuse contenant, d'une part, de 15 à 20 g/l d'un mélange de sels formés à partir des cations sodium, magnésium, calcium, potassium, strontium et bore et des anions chlore, sulfate, carbonate et bicarbonate et, d'autre part, de 3 à 4 % d'acide acétique.

2. Médicament selon la revendication 1, caractérisé en ce que le solution aqueuse renferme, en outre, des composés minéraux azotés choisis entre les nitrites, les nitrates et les sels ammoniacaux.

3. Médicament selon la revendication 1, caractérisé en ce que la solution aqueuse renferme, en outre, des phosphates.

4. Médicament selon la revendication 1, caractérisé en ce que la solution aqueuse renferme, en outre, de la silice.

5. Médicament selon l'une quelconque des revendications 1 à 4, caractérisé en ce que la solution aqueuse est préparée à partir d'eau de mer et de vinaigre blanc.

6. Médicament selon la revendication 5, caractérisé en ce que la solution est stérilisée par filtration à froid sur bougie poreuse.

7. Préparation injectable, caractérisée en ce qu'elle est constituée de la solution aqueuse selon l'une quelconque des revendications 1 à 5, convenablement tamponnée et/ou diluée pour être isotonique au plasma sanguin.

8. Préparation à administrer sous forme de lavement intestinal, caractérisée en ce qu'elle est constituée de la solution selon l'une quelconque des revendications 1 à 6.

9- Utilisation d'une solution aqueuse selon les revendications 1-8 pour la fabrication d'un médicament destiné au traitement des maladies virales, notamment le SIDA.

## Claims

1. New drug comprised of an aqueous solution containing, on the one hand, from 15 to 20 g/l of a mixture of-salts made from cations selected among sodium, magnesium, calcium, potassium, strontium and boron, and from anions selected from chlorine, sulfate, carbonate and bicarbonate, and, on the other hand, from 3 to 4 % of acetic acid.

2. Drug according to claim 1, characterized in that the aqueous solution includes furthermore nitrogen-containing inorganic compounds selected from nitrites, nitrates and ammonia salts.

3 - Drug according to claim 1, characterized in that the aqueous solution includes, furthermore, phosphates.

4 - Drug according to claim 1, characterized in that the aqueous solution includes, furthermore, silica.

5 - Drug according to any one of claims 1 to 4 , characterized in that the aqueous solution is prepared from sea water and white vinegar.

6 - Drug according to claim 1, characterized in that the aqueous solution is sterilized by cold-filtration though a filtering candle.

7 - Injectable preparation, characterized in that it is made from the aqueous solution according to any one of claims 1 to 5, suitably buffered and/or diluted so as to be isotonic to blood plasma.

8 - Preparation for rectal injection, characterized in that it is made from the solution according to any one of claims 1 to 6.

9 - Utilization of yhe aqueous solution according to claims 1 to 8 for making a drug used for treatment of viral diseases, in particular AIDS.

## Patentansprüche

1.- Wässerige Lösung, die einerseits 15 bis 20 g/l ein aus Natrium-, Magnesium-, Kalzium-, Kalium-, Strontium- und Bor-Kationen sowie Chlor-, Sulfat-, Karbonat- und Bikarbonat-Anionen bestehenden Salzgemisch und andererseits 3 bis 4 % Essigsäure enthält.

2 - Arzneimittel entsprechend Anspruch 1, dadurch gekennzeichnet, daß die wässerige Lösung darüber hinaus Stickstoffverbindungen enthält, die unter Nitriten, Nitraten und Ammoniumchloriden ausgewählt werden.

3. Arzneimittel entsprechend Anspruch 1, dadurch gekennzeichnet, daß die wässerige Lösung darüber hinaus Phosphate enthält.

4. Arzneimittel entsprechend Anspruch 1, dadurch gekennzeichnet, daß die wässerige Lösung darüber hinaus Kieselsäure enthält.

5 - Arzneimittel entsprechend eines beliebigen der Ansprüche 1 bis 4, dadurch gekennzeichnet, daß die wässerige Lösung aus Meerwasser und weißem Essig besteht.

6 - Arzneimittel entsprechend Anspruch 5, dadurch gekennzeichnet, daß die Lösung mittels Kaltfiltrierung durch eine Filterkerze sterilisiert wird.

7 - Injizierbares Präparat, dadurch gekennzeichnet, daß es aus einer wässerigen Lösung entsprechend eines beliebigen der Ansprüche 1 bis 5 besteht, und so gepuffert und/oder verdünnt ist, daß es zum Blutplasma isotonisch ist.

8. Als Rektaleinlauf verabreichbares Präparat, dadurch gekennzeichnet, daß es aus der Lösung entsprechend eines beliebigen der Ansprüche 1 bis 6 besteht.

9. Verwendung einer wässerigen Lösung entsprechend Ansprüchen 1 bis 8 zur Herstellung eines für die Behandlung von Viruskrankheiten, insbesondere von AIDS, bestimmten Arzneimittels.